# EUROPEAN PATENT SPECIFICATION

(11) **EP 1 649 805 B1**
(45) Date of publication and mention of the grant of the patent: **25.11.2009**
(21) Application number: 05256472.1
(22) Date of filing: 19.10.2005
(51) Int. Cl.: A61B 5/053

(54) **Electrode belt for the performance of electrodiagnostic procedures**
Elektrodengürtel zur Anwendung in der Elektrodiagnostik
Ceinture d'électrodes pour la réalisation de procédures d'électrodiagnostics

(30) Priority: 20.10.2004 DE 102004050981
(43) Date of publication of application: 26.04.2006
(73) Proprietor: Dräger Medical AG & Co. KG, 23558 Lübeck (DE)
(72) Inventor: Teschner, Eckhard, 21029 Hamburg (DE); Gallus, Thomas, Ratekau (DE); Li, Jianhua, 23558 Lübeck (DE); Poecher, Arndt, 23611 Bad Schwartau (DE); Riggert, Eckhard, 23626 Ratekau (DE); Gärber, Yvo, 23564 Lübeck (DE)
(74) Representative: Greenwood, John David

(56) References cited:
- EP-A- 0 509 689
- DE-U1- 20 220 227
- US-A- 3 805 769
- US-A- 4 121 575
- US-A- 4 263 920
- US-A1- 2002 058 972

## Description

The invention relates to an electrode belt for the performance of electrodiagnostic procedures on the human body.

It has long been known, in connection with various diagnostic procedures, to evaluate electrical signals which are obtained via electrodes applied on the body. The application of electrodes on the body surface required for this must first and foremost guarantee reliability and position stability. For a reliable signal to be obtained, large contact areas are generally sought in order to ensure a good electrical contact.

Two basic principles have essentially become established for the fixing of electrodes.
Either the electrodes are affixed as individually adhering electrodes on the surface of the body or the electrodes are fixed to a carrier means, which ensures a more reliable seating of the electrodes at the desired positions. Numerous different carrier means are known for such fixing of electrodes. General requirements are usually made on the latter, as they are also made on other equipment intended for use in the medical sector. They include, where appropriate, a design as a reusable product and, associated therewith, good suitability for easy cleaning and disinfection or sterilisation. Low production costs, furthermore, are always sought: should the need arise, the possibility of rapid application of such carrier means also needs to be provided, including on recumbent or unconscious patients, and this may possibly have to be undertaken by a single member of the nursing staff.

Furthermore, requirements need to be taken into account that ensure acceptance of the respective carrier means and, therefore, the diagnostic procedure that can be achieved therewith. An attempt is therefore made to find forms of the geometrical arrangement, i.e. a flat design for example, that do not restrict the given patient's capacity for movement or do so as little as possible. In the case of elastic devices, conveniently regulatable expansion characteristics, or ones that are not perceived as troublesome, a surface quality that does not cause discomfort to the patient, as well as convenient wearer characteristics including with long-term applications are desirable. In addition, particular attention needs to be paid to efforts to find arrangements that lead to little or no formation of necroses with the long-term applications.

Various forms of belt, in particular, have become established as carrier means for diagnostic procedures, in which electrodes are arranged essentially in a plane around a patient's body or only individual electrodes have to be affixed to the body.

One such method, which requires the arrangement of several electrodes essentially in a plane around a patient's body, is the method of electroimpedance tomography. Electroimpedance tomography is a method in which, by feeding an electrical alternating current into the human body and measuring the resultant surface potentials at different points of the body, the electrical alternating current impedance between the feed-in and measurement point can be calculated. By means of different combinations of feed-in point and measurement point, for example by successive rotation of the position of the current feed-in point around the body, with simultaneous measurement of the surface potentials along a sectional plane, it is possible by means of suitable mathematical reconstruction algorithms to establish a two-dimensional sectional image of the electrical impedance distribution in the body examined.

A sectional image of the impedance distribution of the human body is of interest in medicine, since the electrical impedance changes both with the content of air as well as with the content of extracellular fluids in the tissue. Both the ventilation, in particular the distribution of air-filled cavities, as well as the perfusion within the sectional image plane can thus be displayed and monitored in a regionally resolved manner. This is of importance especially with thorax examinations.

Since electroimpedance tomography makes relatively high demands on the application of the electrodes, the invention will be explained below taking this method as an example, although transfer of the technical teaching to other electrodiagnostic procedures is possible without problem.

For the clinical acceptance of the method of electroimpedance tomography, the reliable and rapid connection of electrodes to recumbent patients and a durably good contact between the skin and the individual electrodes are of central importance. The use of standard electrodes, i.e. commercially available ECG electrodes for example, belongs to the prior art.

The latter are often connected to electrode cables running individually. In order to suppress electrical interference and marked inductive disturbance, these electrodes are connected in individual cases via shielded lines to the electroimpedance tomography apparatus. This shielding can be actively driven in individual cases. Since electroimpedance tomography is a method with which fed-in signals are first required that must be precisely known in order to enable a meaningful evaluation of received signals, this method obviously exhibits particularly high sensitivity to coupled interference.

Various methods are known for reducing such interference by means of targeted filter algorithms, or more precisely for minimising the same by suitable calibrations with respect to its influence. Since, however, individual cables can also cause interference between one another, relative position stability of the individual cables with respect to one another is absolutely essential for a calibration of this kind. In the case of a large number of cables, this requirement can be met only with considerable expenditure. A fairly large number of individually running cables, moreover, is not very convenient for the patient and the medical staff.

In order to guarantee low susceptibility to error, it is necessary in connection with the use of electrical lines to avoid overloading of these lines. Kinking loads and high tensile loads in particular are to be avoided.

Numerous approaches are known from the prior art which partially overcome the aforementioned problems.

It is known from a generic arrangement to arrange several electrodes on a supporting structure and to control and interrogate the same by means of individual lines which lead to a multi-pole cable. This arrangement, however, is preferably suitable for the performance of ECG examinations. For an application in the area of electroimpedance tomography, this arrangement is too susceptible to faults, since the individual lines are not sufficiently position-stable (WO 97/14346).

It is further known to encapsulate several electrodes with a belt. This, however, presents the manufacturer with considerable difficulties in some cases and reduces subsequent possibilities of adapting such a belt system to special requirements (WO 03/082103 A1).

It is further known to arrange several electrodes displaceably on an elastic band. Under different conditions of use, however, such a solution possibly offers too little safety with respect to the position stability of the electrodes (DE 196 10 246 A1).

It is further known to reduce the susceptibility to faults of a described arrangement with several electrodes by means of special driver circuits. This, however, increases the technical outlay to a not inconsiderable extent and offers only limited safety with regard to the influences of various coupled interferences (DE 101 56 833 A1).

It is further known to increase the informative value of images obtained by electroimpedance tomography by the fact that these images are superimposed with other images which have been obtained by other physical-diagnostic procedures. This, however, increases considerably the expenditure required from the technological standpoint (EP 1000580 A1).

The problem of the invention is to make available an electrode belt which largely avoids the aforementioned drawbacks of the prior art and in particular exhibits good suitability for use in the area of electroimpedance tomography.

US-A-4,121,575 discloses an electrode belt having the features of the preamble of claim 1. The present invention is characterized by the features of the characterizing portion of claim 1. Optional features are recited in the dependent claims.

It is advantageous for each core of the multi-core cable to have a separate shielding.

The embodiment of the core routing inside a multi-care cable guarantees an almost constant position of the individual cores with respect to one another. The influence of various interferences can be effectively reduced in connection with a separate shielding of each core. Such a multi-core cable can be understood us a cable tree consisting of several individually shielded cables, said cable tree being characterised by a particularly good position stability of the individual cables with respect to one another and by a particularly good handling capacity.

The electrode belt according to the invention may have contact means connected to the electrodes which are connected detachably to the connection elements, which are connected in each case to a core of the multi-core cable. In this way, the complete cable structure including the connection elements can be separated from the electrode belt without the electrodes having to be removed from the supporting structure of the belt. Each electrode may have a rigid contact pin, which leads through the belt and projects out of the belt on the side of the belt facing away from the body. Said contact pin may be connected detachably to a suitable connection element. To advantage, the connection takes place in the manner of a press-stud connection. For this purpose, the contact pins of the electrodes can for example have a spherical end on the side of the belt facing away from the body. The connection elements, which are connected in each case to a core of the multi-core cable, have spring elements in back tension, which permit the spherical end of the contact pin to be engaged from behind. The press-stud connection can thus take place simply by pressing-on and pulling-off of the connection elements on the contact pins or can be assisted by means of unlocking aids contained in the connection elements. For this development, use can be made for example of cable systems, such as they are presented in US 2004/0105245 A1. Very reliable results have thus been obtained in a frequency range of 50 - 200 kHz for the feeding-in of the signals. Very small interference levels can be achieved by means of a cable routing arranged in this way, with separate shielding of the individual cores. The inductive disturbance between the cores, moreover, can be readily compensated for, the handling is very user-friendly and movement artifacts due to spacing changes between individual cores are also reduced. The possibility of separating the belt and the cables from one another is an advantage for cleaning measures that are required in everyday use.

For increased wearer comfort, it is particularly advantageous for the electrodes to have a planar surface which is in two-dimensionally extending contact with the body of the test subject and for the edge of the electrodes to end approximately flush with the surface of the belt lying adjacent to the body. A particularly high degree of contact reliability results if the electrodes have a convex surface, which is in two-dimensionally extending contact with the body of the test subject, and if the edge of the electrodes ends approximately flush with the surface of the belt adjacent to the body. As a result of the elevation of the convex surface, a particularly close contact between electrode and body surface results at least in the central region of the electrode surface.

Especially for long-term applications, it is also advantageous for no or only slight skin irritations to occur at the edges of the belt. There is a problem in that such electrode belts are often used with relatively obese patients. The contact pressure of the belt required for a reliable electrical contact can cause the belt to cut relatively deeply into the skin in the case of the latter. In order, however, to avoid skin irritations at the edges of the belt including in the case where it is worn long-term, it is advantageous for the thickness of the belt material to diminish towards the edges. Easier deformability of the belt edges is thus achieved, which prevents overlapping of skin in this region, because the belt can adapt better to the shape of the skin and a sharp-edged termination cannot arise. Skin irritations in the edge region of the electrode belt are avoided particularly effectively when the edges of the belt are designed as a tubular bead. Even with relatively deep penetration of the belt into the patient's skin, the skin is as a result unable to form any folds which have touching edges, but can only shape itself around regions of the tubular bead. It has been shown that such a design of the belt contributes to the efficient avoidance of necroses even in the case of long-term applications.

In order to guarantee protection of the cables against excessive tension the distance between neighbouring electrodes in the unstretched state of the belt are smaller than the length of the multi-core cable between the respective neighbouring connection elements and the multi-core cable has a meandering course approximately parallel to the belt. It has proved to be particularly advantageous for the cable to be approx. 30% longer than the belt in the unstretched state. If the elastic belt is stretched, only the length of the belt changes, the length of the cable remains constant, and the meandering structure in which the cable is routed instead flattens out. With a 30% longer cable routing, therefore, an expansion of the belt of 30% can be achieved without the tensile load on the cable varying. In order to secure the cable against overloading, it is advantageous for the cable also to have a tension relief. This acts as a safeguard against overloading when the belt is stretched to the full length of the cable. The meandering course of the multi-core cable routing, and that the connection elements are connected to the contact means of the electrodes in such a way that they can be rotated about an axis parallel to the normal to the body surface in the region of the two-dimensionally extending contact of the electrodes, enables a rigid clamping of the cable in the connection elements. Kinking of the cable with different tensile loads is thus completely avoided. In this way, long-term stable use is ensured without the need for cable replacement. The quality of the signals is essentially maintained, since sensitive kinks in the cable are avoided.

It is particularly advantageous for the belt and/or the electrodes to be made from material which can be disinfected and sterilised without damage. For this purpose, silicone is provided as the belt and/or electrode material in an advantageous development. In order to maintain the contact properties, moreover, it is advantageous for the electrodes to be made at least partially from conductive or conductively coated plastics material. In an alternative advantageous development, the electrodes are made partly from special steel or from sintered silver chloride.

A particularly high degree of contact and position reliability of the electrodes results if at least individual electrodes have gel pads which are adhesive on one side. In order to facilitate the application of the electrode belt according to the invention, it is advantageous if the latter can be opened at least at one point. It is particularly advantageous for the belt to comprise several individual segments that can be connected to one another and for each of these individual segments to be connected at least to four electrodes. To advantage, these respective four electrodes are connected to connection means, which are connected to different individually shielded cores of a multi-core cable. In this way, the individual segments with electrodes and cable can be separated from one another in the fully assembled state and individually replaced or applied one after the other.

In connection with an application of the electrode belt according to the invention in the chest region, it is advantageous if tensioning means are additionally included, which ensure a rigid seating of individual electrodes in the concave areas of the body surface. This tensioning means can be designed in such a way that at least one gel cushion is included, which enables a support of the electrodes at a tensioning means, for example a belt, arranged at a distance in front of the body.

A particularly effective shielding with respect to interference can be achieved if the individual cores of the multi-core cable are each double-shielded. In another particularly effective development, the individual cores of the multi-core cable each have an individual shield and are in addition surrounded as a whole by a second common shield. Individual shields or all shields can be actively driven or act passively.

A particularly high degree of functional reliability can be achieved if the connection between the belt and the connection elements is designed in such a way that the penetration of fluids into the region in which the electrical contact is produced is avoided or at least made difficult. This can be achieved, for example, by forming seals on the belt, which engage in a keyed manner in a groove in the body of the individual connection elements.

The invention will be explained in greater detail using an example of embodiment.

The respective figures show the following:
- Figure 1: a sectional representation of an electrode belt according to the invention with electrodes with a planar contact surface,
- Figure 2: a sectional representation of an electrode belt according to the invention with electrodes with a convex contact surface,
- Figure 3: a diagrammatic overall representation of an electrode belt according to the invention,
- Figure 4: the representation of an electrode belt according to the invention in the relaxed state,
- Figure 5: the representation of electrode belt according to the invention in the stretched state,
- Figure 6: a sectional representation of a particularly advantageous form of belt,
- Figure 7: a diagrammatic representation of an electrode belt according to the invention with a gel cushion for supporting the electrodes in the region of the breastbone,
- Figure 8: a diagrammatic representation of a connection element with an electrode affixed thereto,
- Figure 9: a diagrammatic representation of a connection element with an electrode affixed thereto, whereby the region of the electrical contact is protected against the penetration of fluids.

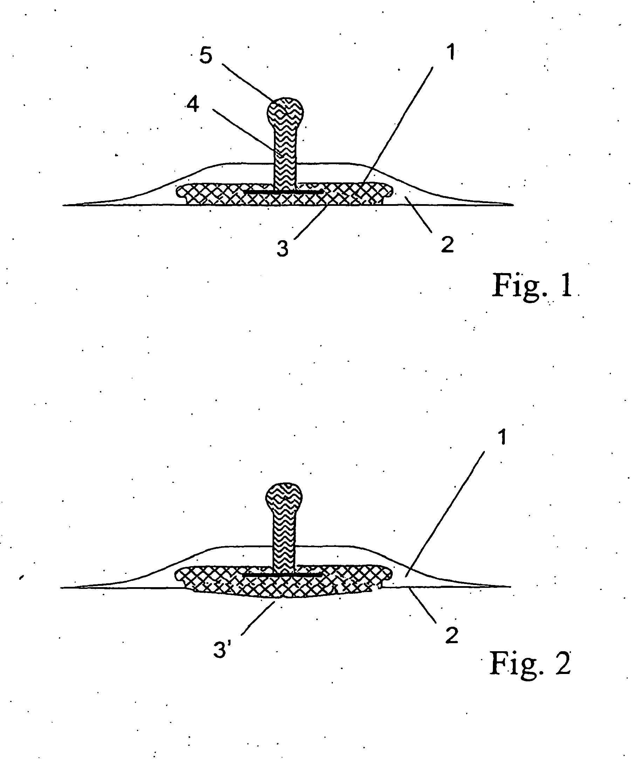

Figure 1 shows a sectional representation of an electrode belt according to the invention, and how an electrode 1 is integrated into an elastic belt 2. Electrode 1 has a planar contact surface 3. This terminates flush with belt 2, so that a uniform even surface is formed at the patient's body. A contact pin 4 leads through belt 2, projects out of belt 2 on the side facing away from the body and has a spherical end 5. This spherical end 5 can be introduced into a suitable connection element using the press-stud principle.

By means of this press-stud connection, an attachment of the connection elements can be achieved particularly advantageously, wherein the connection elements are fixed at a position, but are mounted so as to be rotatable about an axis normal to the electrode surface. The thickness of the belt material diminishes from the middle to the edge, which gives rise to enhanced wearer comfort. Overall, this embodiment enables a very flat design.

Figure 2 shows a similar structure according to the invention with an electrode 1, which has a convex contact surface 3'. Contact surface 3' thus projects slightly from belt 2, which ensures a particularly effective contact with the patient's skin. In addition, whole contact surface 3' projects slightly above the belt material. This projection is dimensioned in such a way that there is no damage to the skin and no loss of wearer comfort. The edge regions of belt 2 running away flat also ensure in the case of obese patients and relatively high contact pressures that skin irritation at the edge of the belt does not occur when the belt cuts in. The regions of the belt tapering in flat adapt as required to the shape of any skin fold.

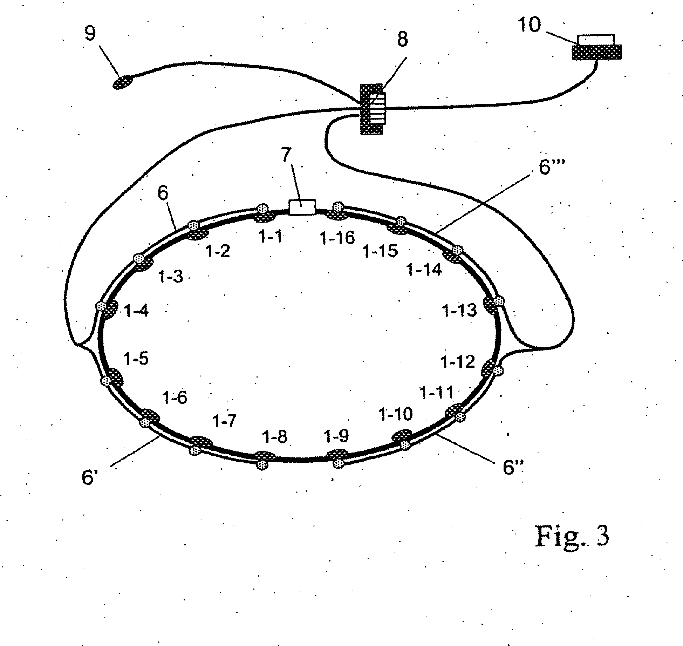

Figure 3 shows a diagrammatic overall representation of an electrode belt according to the invention, which surrounds the upper body of a patient. It comprises a belt 2 made from an expandable biocompatible material such as silicone, which exhibits good expansion behaviour with little increase in force and causes scarcely any allergenic stresses. In the present case, the electrode belt further comprises 16 rigidly integrated electrodes 1-1 to 1-16 made from silicone, which are connected to the cable tree by means of a press-stud connection on the rear side of the belt. This cable tree essentially comprises one or more multi-core cables, the individual cables whereof are each shielded individually. In the present example of embodiment, the arrangement includes two electrode groups with eight electrodes in each case, which are supplied from two directions with corresponding cable connections, whereby in each case four electrodes are connected via connection elements respectively to a common cable 6, 6', 6", 6'''. At intermediate points between the electrode groups, in this case between electrodes 1-1 and 1-16, a separation of the electrode belt can to advantage be undertaken at a fastening element 7 in order to facilitate the applicability. Apart from the fixed integration of electrodes into the belt material, a detachable connection between electrodes and belt, for example by insertion of electrodes into a belt provided with prepared holes, is possible in a further advantageous development. As a result, particularly straightforward cleaning and disinfection can be carried out, or adaptation of the overall electrode belt to changed requirements. As a result of the elasticity of the belt material, a contact pressure dependent on the thorax circumference and the belt length is exerted on the electrodes. The four multi-core cables 6, 6', 6", 6"' are led at the side of the patient, in each case in pairs, to a plug-in connection 8 close to the patient, to which a reference electrode 9 and a connection cable 10 for connection to an electroimpedance tomography apparatus are connected.

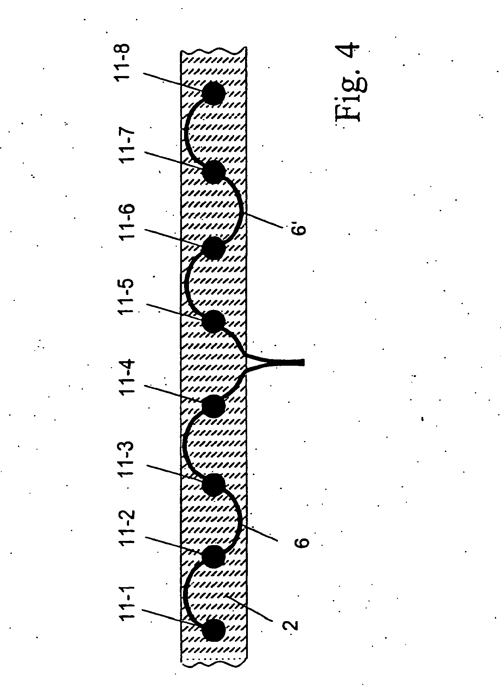

Figure 4 shows the side of a half of an electrode belt represented in figure 3, said side facing away from the body. The eight electrodes are supplied by two multi-core cables 6, 6', whereby four electrodes are connected via connection elements 11-1 to 11-4 and 11-5 to 11-8 in each case to one and the same multi-core cable and the connection elements are each connected to another individually shielded core. Belt 2 is in the relaxed state. The length of the multi-core cable is roughly 30% greater than the length of supporting belt 2 in the relaxed state. Connection elements 11-1 to 11-8 are mounted rotatably and have an orientation which allows the multi-core cables to have a kink-free meandering course.

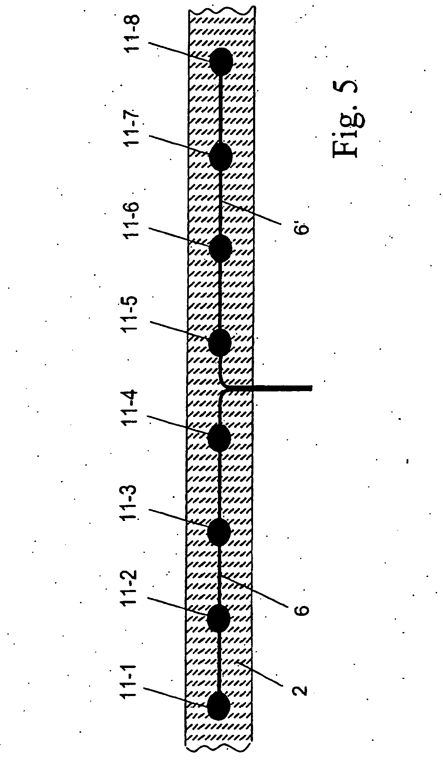

Figure 5 shows the same detail of an electrode belt according to the invention in the state overstretched by 30%. In this situation, belt 2 and multi-core cables 6, 6' run approximately in parallel. Rotatably mounted connection elements 11-1 to 11-8 are swivelled into a position which enables the parallel course of the cable kink-free in front of the belt. In this overstretched state, moreover, a tension relief integrated into the multi-core cable takes effect. Further stretching is not possible, since the multi-core cable connected via the connection elements and the electrodes to the belt now acts as a stop.

The use of a multi-core cable with which every core is individually shielded brings technological advantages. Such cables can be manufactured as products sold by the metre and are separated at the required points only when application takes place on an electrode belt according to the invention, whereby only the core that is to be connected to the corresponding connection element is actually separated electrically in such separation, whilst the other remaining cores run past the connection point without damage to the shield or the core. All the cores of the multi-core cable thus run over the same length of the multi-core cable, whereby each core is interrupted once at another point. This configuration also opens up other possibilities of actively driven shielding or shielding that takes place passively. The individual shields around the cores can additionally be combined with other variants of the shielding.

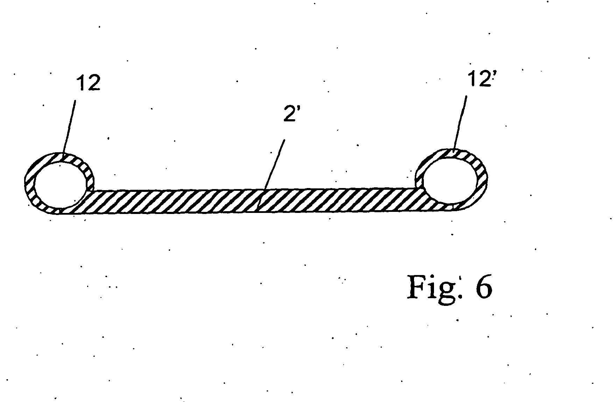

Figure 6 shows a further development of an electrode belt according to the invention in a diagrammatic sectional representation of carrying belt 2' without electrodes. Its edge regions are designed as a tubular bead 12, 12'. This bead prevents the formation of sharp skin folds and thus effectively counteracts skin irritations or necroses, even with long-term application. In a particularly advantageous development, these tubular beads 12, 12' can additionally be filled with a gas, for example air, which allows adjustment of the diameter of the tubular beads. The wearer properties of the electrode belt according to the invention can thus be adapted in terms of its wearer comfort to the requirements of different patients. In a simplified form, the development as an elastic circular bead without a cavity or the possibility of filling is also provided for.

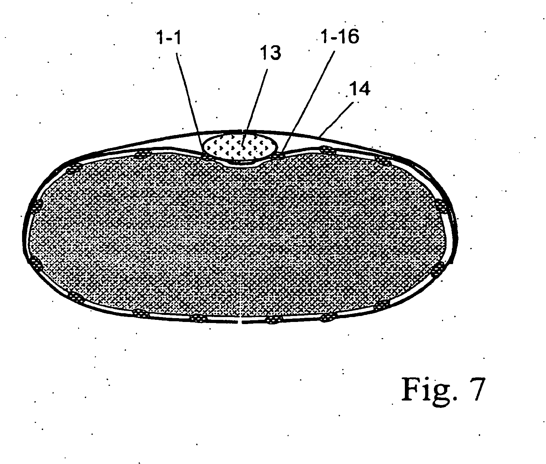

Figure 7 shows a diagrammatic representation of an electrode belt according to the invention with a gel cushion 13 for supporting the electrodes in the region of the breastbone. The electrode belt surrounds the whole upper body of a patient. In the region of the breastbone, the upper body has a concave area in which, without aid, the electrodes would not have any contact with the skin when the belt is stretched tight. For this reason, a belt-like supporting means 14 is included, which spans the concave area of the upper body. Gel cushion 13, which represents a flexible spacer, can rest against this supporting means. The required contact pressure on electrodes 1-1 and 1-16 can thus be exerted via gel cushion 13 in the concave area of the upper body.

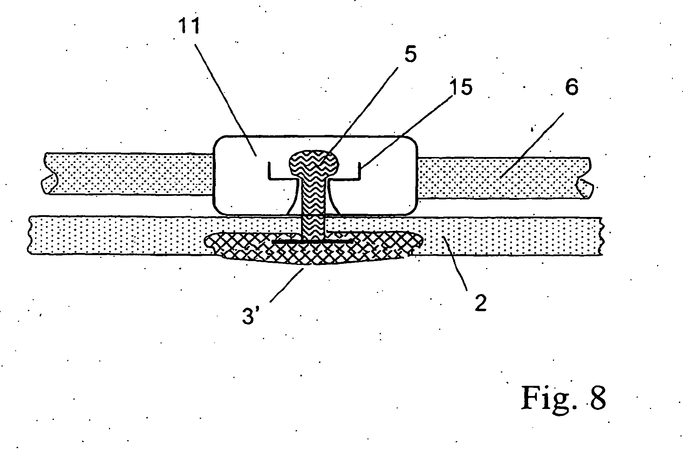

Figure 8 shows a diagrammatic representation of a connection element 11 with an electrode with a convex contact surface 3' affixed thereto. The electrode is embedded in an elastic belt 2. Connection element 11 is connected to a multi-core cable 6. Arranged in the interior of the connection element are spring elements 15, which engage spherical end 5 of the contact pin from behind and thus make for a press-stud connection between electrode 1 and connection element 11. Since spring elements 15 can slide around the contact pin, they enable a rotary mounting of contact element 11, whereby the rotation essentially takes place about the main axis of the contact pin.

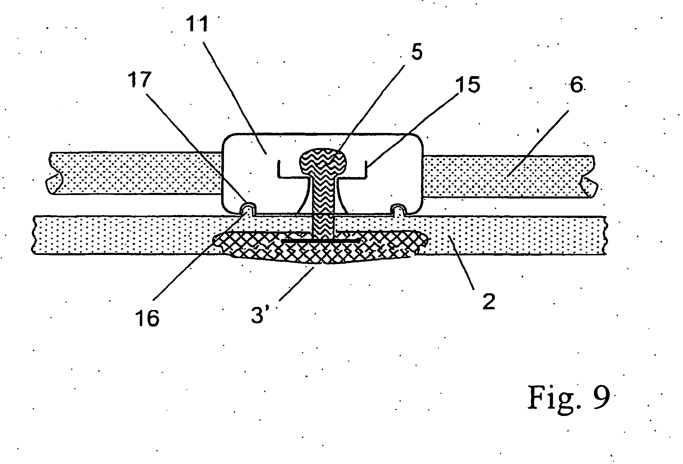

Figure 9 shows a diagrammatic representation of a contact element 11 with an electrode affixed thereto, whereby the region of the electrical contact is secured against the penetration of fluids. Sealing bead 16 is formed on the elastic belt 2 in the contact region with connection element 11. The body of connection element 11 has a groove 17 complementary to this sealing bead 16. The sealing bead engages in a keyed manner in groove 17. The connection, however, remains rotatable. In this way, all the advantageous developments of the invention can be used in combination with particularly reliable contacting.

## Claims

1. An electrode belt for the performance of electrodiagnostic procedures on the human body, comprising:
a belt, which is made at least partially from an elastic material;
several electrodes connected mechanically to the belt (1), said electrodes being in two-dimensionally extending contact with the body of the test subject;
at least one contact means leading through the belt from the electrodes (1) and connected to a connection element (11); the belt being **characterized in that**:
the at least one contact means are connected in each case to a core of a multi-core cable (6):
the multi-core cable has a meandering course approximately parallel to the belt;
the connection elements (11) are connected to the contact means of the electrodes (1) in such a way that said connection elements can be rotated about an axis parallel to the normal of the belt surface in the region of the two-dimensionally extending contact of the electrodes; and
the distance between neighbouring electrodes (1) in the unstretched state of the belt is smaller than the length of the multi-core cable (6) between the respective neighbouring connection elements.

2. The electrode belt according to claim 1, in which each core of the multi-core cable (6) has a separate shielding.

3. The electrode belt according to claim 1 or 2, in which the contact means are connected detachably to the connection elements (11), which are each connected to a core of the multi-core cable (6).

4. The electrode belt according to claim 3, in which the electrodes (1) each have a rigid contact pin (4) which leads through the belt, projects out of the belt on the side of the belt facing away from the body and is connected detachably to the corresponding connection element (11).

5. The electrode belt according to claim 3 or 4, in which the contact pins (4) have a spherical end (5) on the side of the belt facing away from the body, and, optionally, in which the connection elements (11) have spring elements (15) which permit the spherical end (5) of the contact pins (4) to be engaged from behind.

6. The electrode belt according to any preceding claim, in which the electrodes (1) have a planar surface (3) which is in two-dimensionally extending contact with the body of the test subject and the edge of the electrodes ends approximately flush with the surface of the belt adjacent to the body.

7. The electrode belt according to any preceding claim, in which the electrodes (1) have a convex surface (3') which is in two-dimensionally extending contact with the body of the test subject and the edge of the electrodes ends approximately flush with the surface of the belt adjacent to the body.

8. The electrode belt according to any preceding claim, in which the thickness of the electrode belt diminishes towards the edges, or in which the edges of the belt are formed as a tubular bead (12, 12').

9. The electrode belt according to any preceding claim, in which the multi-core cable has a tension relief.

10. The electrode belt according to any preceding claim, in which the belt and/or the electrodes are made from material which can be disinfected and sterilised without damage, and, optionally, in which any of:
a) the belt is made at least partially from silicone;
b) the electrodes are made at least partially from silicone;
c) the electrodes are made at least partially from conductive or conductively coated plastics material;
d) the electrodes are made at least partially from special steel; and
e) the electrodes are made at least partially from sintered silver chloride.

11. The electrode belt according to any one preceding claim, in which gel pads adhesive on one side for improving the skin contact are applied on at least several electrodes.

12. The electrode belt according to any preceding claims, in which the belt can be opened at least at one point.

13. The electrode belt according to any preceding claim, in which the belt comprises several individual segments that can be connected to one another and each of these individual segments is connected at least to four electrodes.

14. The electrode belt according to any preceding claim, in which at least four electrodes are connected in each case to connection means, which are connected to different individually shielded cores of one and the same multi-core cable.

15. The electrode belt according to any preceding claim, in which tensioning means are included which ensure a rigid seating of individual electrodes in the concave areas of the body surface, and, optionally the tensioning means comprises at least one gel cushion (13).

16. The electrode belt according to any preceding claim, in which the individual cores of the multi-core cable are each double-shielded, or the individual cores of the multi-core cable are each individually shielded and surrounded by a second common shield.

17. The electrode belt according to any preceding claim, in which the connection between the belt and the connection elements is designed in such a way that penetration of fluids into the region in which the electrical contact is produced is avoided or at least made difficult, and, optionally, seals (16) are formed on the elastic belt, which seals engage in a keyed manner in a groove (17) in the body of the individual connection elements (11).

## Patentansprüche

1. Elektrodengürtel zur Anwendung in der Elektrodiagnostik am menschlichen Körper, bestehend aus
einem Gürtel, der zumindest teilweise von einem elastischen Material gebildet ist, mehreren mechanisch mit dem Gürtel (1) verbundenen Elektroden, wobei die Elektroden in einem zweidimensional verlaufenden Kontakt mit dem Körper der Testperson stehen, und
zumindest einem durch den Gürtel von den Elektroden (1) führenden und mit einem Verbindungselement (11) verbundenen Kontaktmittel, wobei der Gürtel **dadurch gekennzeichnet ist, dass**
das zumindest eine Kontaktmittel jeweils mit einer Ader eines mehradrigen Kabels (6) verbunden ist,
das mehradrige Kabel einen mäanderförmigen Verlauf in etwa parallel zum Gürtel aufweist,
die Verbindungselemente (11) mit dem Kontaktmittel der Elektroden (1) derart verbunden sind, dass die Verbindungselemente um eine Achse parallel zur Normalen der Gürteloberfläche im Bereich des zweidimensional verlaufenden Kontakts der Elektroden gedreht werden können, und
der Abstand zwischen benachbarten Elektroden (1) im ungestreckten Zustand des Gürtels kleiner ist als die Länge des mehradrigen Kabels (6) zwischen den jeweiligen benachbarten Verbindungselementen.

2. Elektrodengürtel nach Anspruch 1, bei dem jede Ader des mehradrigen Kabels (6) eine gesonderte Abschirmung besitzt.

3. Elektrodengürtel nach Anspruch 1 oder 2, bei dem die Kontaktmittel lösbar mit den Verbindungselementen (11) verbunden sind, die jeweils mit einer Ader des mehradrigen Kabels (6) verbunden sind.

4. Elektrodengürtel nach Anspruch 3, bei dem die Elektroden (1) jeweils einen steifen Kontaktstift (4) aufweisen, der durch den Gürtel hindurchgeführt ist, aus dem Gürtel auf der vom Körper fortweisenden Seite des Gürtels vorsteht und lösbar mit dem entsprechenden Kontaktelement (11) verbunden ist.

5. Elektrodengürtel nach Anspruch 3 oder 4, bei dem die Kontaktstifte (4) ein kugeliges Ende (5) auf der vom Körper fortweisenden Seite des Gürtels aufweisen und bei dem, wahlweise, die Verbindungselemente (11) Federelemente (15) besitzen, die einen Eingriff mit dem kugeligen Ende (5) der Kompaktstifte (4) von hinten ermöglichen.

6. Elektroden nach einem vorhergehenden Anspruch, bei dem die Elektroden (1) eine eine ebene Oberfläche (3) besitzen, die in zweidimensional verlaufendem Kontakt mit dem Körper der Testperson steht, und der Rand der Elektroden in etwa bündig mit der Oberfläche des Gürtels angrenzend an den Körper ausläuft.

7. Elektrodengürtel nach einem vorhergehenden Anspruch, bei dem die Elektroden (1) eine konvexe Oberfläche (3') besitzen, die in zweidimensional verlaufendem Kontakt mit dem Körper der Testperson steht, und der Rand der Elektroden in etwa bündig mit der Oberfläche des Gürtels angrenzend an den Körper ausläuft.

8. Elektrodengürtel nach einem vorhergehenden Anspruch, bei dem sich die Dicke des Elektrodengürtels zu den Rändern hin verringert oder bei dem die Ränder des Gürtels als rohrförmige Wulst (12,12') ausgebildet sind.

9. Elektrodengürtel nach einem vorhergehenden Anspruch, bei dem das mehradrige Kabel eine Zugentlastung besitzt.

10. Elektrodengürtel nach einem vorhergehenden Anspruch, bei dem der Gürtel und /oder die Elektroden von einem Material gebildet sind, das schadlos desinfiziert und sterilisiert werden kann, und, wahlweise, mit einem der Merkmale:
a) der Gürtel ist zumindest teilweise von Silicon gebildet,
b) die Elektroden sind zumindest teilweise von Silicon gebildet,
c) die Elektroden sind zumindest teilweise von leitendem oder leitend beschichtetem Kunststoffmaterial gebildet,
d) die Elektroden sind zumindest teilweise von Edelstahl gebildet und
e) die Elektroden sind zumindest teilweise von gesintertem Silberchlorid gebildet.

11. Elektrodengürtel nach einem vorhergehenden Anspruch, bei dem auf einer Seite haftende Gelkissen zum Verbessern des Hautkontakts an zumindest mehreren Elektroden angebracht sind.

12. Elektrodengürtel nach einem vorhergehenden Anspruch, bei dem der Gürtel an zumindest einem Punkt geöffnet werden kann.

13. Elektrodengürtel nach einem vorhergehenden Anspruch, bei dem der Gürtel mehrere einzelne Segmente umfasst, die miteinander verbunden werden können und wobei jedes dieser einzelnen Segmente mit zumindest vier Elektroden verbunden ist.

14. Elektrodengürtel nach einem vorhergehenden Anspruch, bei dem zumindest vier Elektroden jeweils mit Verbindungsmitteln verbunden sind, die mit verschiedenen einzeln abgeschirmten Adern ein und desselben mehradrigen Kabels verbunden sind.

15. Elektrodengürtel nach einem vorhergehenden Anspruch, bei dem Spannmittel vorhanden sind, die einen strammen Sitz einzelner Elektroden in konkaven Bereichen der Körperoberfläche gewährleisten und bei dem wahlweise die Spannmittel zumindest ein Gelkissen (13) umfassen.

16. Elektrodengürtel nach einem vorhergehenden Anspruch, bei dem die einzelnen Adern des mehradrigen Kabels doppelt abgeschirmt sind oder bei dem die einzelnen Adern des mehradrigen Kabels jeweils einzeln abgeschirmt und von einer zweiten gemeinsamen Abschirmung umgeben sind.

17. Elektrodengürtel nach einem vorhergehenden Anspruch, bei dem die Verbindung zwischen dem Gürtel und den Verbindungselementen so ausgebildet ist, dass ein Eindringen von Fluids in den Bereich, in dem der elektrische Kontakt hergestellt wird, vermieden oder zumindest erschwert ist, und, wahlweise, Abdichtungen (16) am elastischen Gürtel gebildet sind, welche Abdichtungen nach Art einer Feder in eine Nut (17) im Gehäuse der einzelnen Verbindungselemente (11) eingreifen.

## Revendications

1. Ceinture d'électrodes pour la réalisation de procédures d'électrodiagnostics sur le corps humain, comprenant :
une ceinture, qui est constituée au moyen partiellement d'un matériau élastique ;
plusieurs électrodes reliées mécaniquement à la ceinture (1), lesdites électrodes formant un contact s'étendant bidimensionnellement avec le corps du sujet de l'essai ; au moins un moyen de contact passant à travers la ceinture depuis les électrodes (1) et relié à un élément de connexion (11) ; la ceinture étant **caractérisée en ce que** :
l'au moins un moyen de contact est relié dans chaque cas à un conducteur d'un câble multiconducteur (6) ;
le câble multiconducteur présente un trajet en dents de scie à peu près parallèle à la ceinture ;
les éléments de connexion (11) sont reliés aux moyens de contact des électrodes (1) de telle manière que lesdits éléments de connexion puissent être tournés autour d'un axe parallèle à la perpendiculaire de la surface de la ceinture dans la région du contact s'étendant bidimensionnellement des électrodes ; et
la distance entre les électrodes voisines (1) dans l'état détendu de la ceinture est inférieure à la longueur du câble multiconducteur (6) entre les éléments de connexion voisins respectifs.

2. Ceinture d'électrodes selon la revendication 1, dans laquelle chaque conducteur du câble multiconducteur (6) a un blindage distinct.

3. Ceinture d'électrodes selon la revendication 1 ou 2, dans laquelle les moyens de contact sont reliés de façon détachable aux éléments de connexion (11), qui sont chacun reliés à un conducteur du câble multiconducteur (6).

4. Ceinture d'électrodes selon la revendication 3, dans laquelle les électrodes (1) ont chacune un axe de contact rigide (4) qui passe à travers la ceinture, fait saillie de la ceinture sur le côté de la ceinture orienté à l'opposé du corps et est relié de façon détachable à l'élément de connexion (11) correspondant.

5. Ceinture d'électrodes selon la revendication 3 ou 4, dans laquelle les axes de contact (4) ont une extrémité sphérique (5) sur le côté de la ceinture orienté à l'opposé du corps et, éventuellement, dans laquelle les éléments de connexion (11) ont des éléments de ressort (15) qui permettent à l'extrémité sphérique (5) des axes de contact (4) d'être mise en prise depuis l'arrière.

6. Ceinture d'électrodes selon l'une quelconque des revendications précédentes, dans laquelle les électrodes (1) ont une surface plane (3) qui forme un contact s'étendant bidimensionnellement avec le corps du sujet de l'essai et le bord des extrémités d'électrodes vient en affleurement approximatif avec la surface de la ceinture adjacente au corps.

7. Ceinture d'électrodes selon l'une quelconque des revendications précédentes, dans laquelle les électrodes (1) ont une surface convexe (3') qui forme un contact s'étendant bidimensionnellement avec le corps du sujet de l'essai et le bord des extrémités d'électrode vient en affleurement approximatif avec la surface de la ceinture adjacente au corps.

8. Ceinture d'électrodes selon l'une quelconque des revendications précédentes, dans laquelle l'épaisseur de la ceinture d'électrodes diminue vers les bords, ou dans laquelle les bords de la ceinture d'électrodes sont formés comme un bourrelet tubulaire (12, 12').

9. Ceinture d'électrodes selon l'une quelconque des revendications précédentes, dans laquelle le câble multiconducteur présente un relâchement de tension.

10. Ceinture d'électrodes selon l'une quelconque des revendications précédentes, dans laquelle la ceinture et/ou les électrodes sont constituées d'un matériau qui peut être désinfecté et stérilisé sans s'endommager, et, éventuellement, dans laquelle :
a) la ceinture est constituée au moins partiellement de silicone ;
b) les électrodes sont constituées au moins partiellement de silicone ;
c) les électrodes sont constituées au moins partiellement de matériau plastique conducteur ou revêtu conductivement ;
d) les électrodes sont constituées au moins partiellement d'acier spécial ; et
e) les électrodes sont constituées au moins partiellement de chlorure d'argent fritté.

11. Ceinture d'électrodes selon l'une quelconque des revendications précédentes, dans laquelle des tampons de gel adhésifs sur un côté pour améliorer le contact avec la peau sont appliqués sur au moins plusieurs électrodes.

12. Ceinture d'électrodes selon l'une quelconque des revendications précédentes, dans laquelle la ceinture peut être ouverte au moins en un point.

13. Ceinture d'électrodes selon l'une quelconque des revendications précédentes, dans laquelle la ceinture comprend plusieurs segments individuels qui peuvent être reliés l'un à l'autre et chacun de ces segments individuels est relié à au moins quatre électrodes.

14. Ceinture d'électrodes selon l'une quelconque des revendications précédentes, dans laquelle au moins quatre électrodes sont reliées dans chaque cas à des moyens de connexion, qui sont reliés à différents conducteurs blindés individuellement d'un seul et même câble multiconducteur.

15. Ceinture d'électrodes selon l'une quelconque des revendications précédentes, dans laquelle sont inclus des moyens de tension pour assurer une assise rigide des électrodes individuelles dans les zones concaves de la surface corporelle, et, les moyens de tension comprennent éventuellement au moins un coussin de gel (13).

16. Ceinture d'électrodes selon l'une quelconque des revendications précédentes, dans laquelle les conducteurs individuels du câble multiconducteur sont chacun doublement blindés, ou les conducteurs individuels du câble multiconducteur sont chacun blindés individuellement et entourés par un second blindage commun.

17. Ceinture d'électrodes selon l'une quelconque des revendications précédentes, dans laquelle la connexion entre la ceinture et les éléments de connexion est conçue de telle manière que la pénétration de fluides dans la région dans laquelle le contact électrique est produit est évitée ou du moins rendue difficile, et, éventuellement, des joints (16) sont formés sur la ceinture élastique, lesquels joints se mettent en prise d'une manière clavetée dans une rainure (17) dans le corps des éléments de connexion individuels (11).
